# EUROPEAN PATENT SPECIFICATION

(11) **EP 1 072 676 B1**
(45) Date of publication and mention of the grant of the patent: **01.09.2010**
(21) Application number: 99913660.9
(22) Date of filing: 14.04.1999
(51) Int. Cl.: C12N 1/14, C12N 1/16, C12N 1/38, A23L 1/227

(54) **MICROBIAL CULTURE WITH ENHANCED GLUTAMINASE ACTIVITY AND UTILIZATION THEREOF**
MIKROBENKULTUR MIT ERHÖHTER GLUTAMINASE-AKTIVITÄT UND IHRE VERWENDUNG
CULTURE MICROBIENNE AVEC ACTIVITE DE LA GLUTAMINASE AMELIOREE ET SON UTILISATION

(30) Priority: 16.04.1998 JP 12162198
(43) Date of publication of application: 31.01.2001
(73) Proprietor: Ajinomoto Co., Inc., Tokyo 104-8315 (JP)
(72) Inventor: YUASA, Ari Ajinomoto Co., Inc., Kawasaki-shi Kanagawa 210-0801 (JP); OKAMURA, Hideki Ajinomoto Co., Inc., Kawasaki-shi Kanagawa 210-0801 (JP); KATAOKA, Jiro Ajinomoto Co., Inc., Kawasaki-shi Kanagawa 210-0801 (JP)
(74) Representative: Strehl Schübel-Hopf & Partner
(86) International application number: PCT/JP1999/001983
(87) International publication number: WO 1999/054438

(56) References cited:
- GB-A- 1 199 068
- JP-A- 7 099 923
- JP-A- 7 184 634
- JP-A- 59 173 090
- JP-A- 62 239 966
- JP-B1- 41 005 909
- JP-B1- 50 025 037
- JONES, F.N.H. ET AL: "The production and optimisation of glutaminase from aspergillus awamori" ABSTRACTS OF THE GENERAL MEETING OF THE AMERICAN SOCIETY OF MICROBIOLOGY, vol. 95, no. 0, 1995, page 369 XP008034126 WASHINGTON, DC, USA
- COOK W.R. et al., "Occurence of an Inducible Glutaminase in Bacillus Licheniformis", J. OF BACTERIOLOGY, Oct. (1981),Vol. 148, No. 1, pages 365-367, XP002919387

## Description

### Technical Field

The present invention relates to a process for producing a microbial culture having an increased glutaminase activity. More specifically, it relates to a process for producing a microbial culture having an increased glutaminase activity by releasing catabolite repression during the incubation of a microorganism capable of producing glutaminase and feeding a nitrogen source in the intermediate stage of the incubation as required.

Further, the present invention relates to a microbial culture having an increased glutaminase activity, a process for producing hydrolyzed protein using the microbial culture, and hydrolyzed protein obtained by this process. Since this hydrolyzed protein has a high glutamic acid content, it exhibits a strong seasoning power, and is quite valuable as a seasoning.

### Background Art

A glutaminase is an enzyme widely distributed in the biological world, and it acts on L-glutamine whereby an amide group is hydrolyzed to form L-glutamic acid and ammonia.

Monosodium L-glutamate obtained by neutralizing L-glutamic acid with sodium hydroxide or sodium carbonate is a basic substance of all "umami" tastes. This substance is used most in seasonings, and it is an additive which is dispensable not only for domestic foods but also for the production of processed foods.

The production of naturally-derived food seasonings which have been so far known is mainly conducted through hydrolysis of proteins. Among such seasonings, hydrolyzed proteins include acid-hydrolyzed protein and enzyme-hydrolyzed protein. Acid-hydrolyzed protein includes hydrolyzed vegetable protein obtained by using vegetable protein from soybeans, wheat, etc., as a starting material and hydrolyzed animal protein obtained by using animal protein such as gelatin and milk casein as a starting material. The composition of amino acids as the main ingredient varies depending on the starting material, and influences a taste, a sweetness and the like. For example, in a hydrolyzate solution obtained by hydrolysis of de-fatted soybeans with hydrochloric acid, a hydrolysis rate (formol-type nitrogen/total nitrogen) is as high as 70 % or more, and a rate of liberation of glutamic acid (glutamic acid/total nitrogen) is as high as 1.2. Thus, it is quite excellent in view of the umami.

However, when the hydrolyzed protein is obtained by the hydrolysis with hydrochloric acid, it requires a reaction from 1 to 2 days at 100 °C. Such a reaction at the high temperature for the long period of time consumes a large amount of energy. Further, the hydrolysis of the protein with the acid is simple, but it involves defects such as occurrence of an offensive smell, excessive hydrolysis of amino acids and a high salt content for neutralization.

Besides, in recent years, it has been pointed out that chlorohydrins such as monochloropropanols and dichloropropanols which are harmful to the human body are formed in the acid hydrolysis. Accordingly, this method is getting problematic in view of food material use.

Meanwhile, many approaches have been attempted with respect to a process for enzymatically producing a solution of amino acids. A conjugated enzyme system is required for hydrolysis of a complicated substrate into amino acids, and a koji mold, mainly Aspergillus oryzae, is ordinarily used to achieve the object. As a typical amino acid liquid produced by this process, soy sauce is mentioned. Soy sauce is produced by heat-treating a protein starting material, propagating a koji mold thereon, and then fermenting and aging the resulting substance in salt water.

Although this method takes much labor and time, it has defects that a rate of liberation of amino acids is low and that especially a rate of liberation of glutamic acid which is contained in the largest amount in soybean protein and which is important as a seasoning property is low.

Thus, in recent years, seasonings which are produced by the enzymatic hydrolysis of proteins have been increasingly required chiefly in Europe. However, seasonings free from monochlorohydroxypropanols and dichloropropanols and having a strong umami equal to that obtained by hydrolysis with hydrochloric acid have not existed.

In the production of soy sauce, the amount of glutamic acid is insufficient because of the shortage of the glutaminase activity of a koji mold. Accordingly, in the solid culture, breeding of a strain having a high activity by cell fusion of a strain having a high protease activity and a strain having a high glutaminase activity has been conducted (S. Ushijima, T. Nakadai: Agric. Biol. Chem., 51 (4), 1051 (1987); S. Ushijima, T. Nakadai, K. Uchida: Agric. Biol. Chem., 51 (10), 2781 (1987): S. Ushijima, T. Nakadai, K. Uchida: Nippon Shoyu Kenkyusyo Zasshi, 17, (3), 89 (1991); JP-B-3-73271; JP-B-3-68672).

Further, it has been known that a large number of enzyme systems undergo catabolite repression, that is, biosynthesis of the enzymes is repressed by a carbon source which is rapidly consumed (Aunstrp, K. et al.: "Microbial Technology", vol. 1 (2nd edition), Academic Press, New York, 282 (1979)). In Aspergillus nidulans, studies on the enzymes involved in proline metabolism or alcohol utilization have been conducted. In Aspergillus oryzae, studies on Taka-amylase gene expression control have been conducted. CreA protein which is a CreA gene product has been known to conduct negative regulation of synthesis of various enzymes (N. Tsukakoshi: Chemistry and Biology, 32(1), 48 (1994)).

Nevertheless, in Aspergillus nidulans, it is only reported that when a nitrogen source for the incubation is ammonium, the production of glutaminase is repressed, and there is not the least report stating that a glutaminase of a koji mold or a yeast undergoes catabolite repression. Further, with respect to a glutaminase in the liquid culture of strains, there are only the following reports:
Improvement of an amount of glutaminase produced in cells by the addition of monopotassium phosphate in the liquid culture of Aspergillus oryzae and Aspergillus sojae (T. Yamazaki, K. Inamori, I. Uchida: Nippon Shoyu Kenkyusyo Zasshi, 22, (1), 13 (1996));
Induction of glutaminase by glutamine and inhibition by glucose in Bacillus licheniformis (Cook, William R., Hoffman. Joshua H, Bernlohr. Robert W.: J. Bacteriol., 148 (1), 365 (1981));
Induction of glutaminase by glutamine in Pseudomonas boreopolis (Berezov, T. T., Khisamov, G. Z., Evseev, L. P., Zanin, V. A.: Byull. Eksp. Biol. Med., 76 (10), 54 (1973));
Inhibition of glutaminase production by glutamine in Escherichia coli (Varricchio, Frederick: Arch. Mikrobiol., 81(3), 234 (1972)); and
Decrease of glutaminase production by the addition of citric acid, succinic acid and α-ketoglutaric acid in Pseudomonas fluorescens (Nikolaev, A. Ya., Sokolov, N.N., Mardashev, S. R.: Biokhimiya. 36 (3), 643 (1971)).

As stated above, there is almost no report with respect to a method of improving glutaminase production with fungi including microorganisms belonging to the genus Aspergillus and yeasts.

Jones, F.N.H. et al.: "The production and optimisation of glutaminase from Aspergillus awamori", ABSTRACTS OF THE GENERAL MEETING OF THE AMERICAN SOCIETY OF MICROBIOLOGY, vol. 95, no. 0, 1995, page 369 discloses the production and optimisation of glutaminase from Aspergillus awamori and shows that glucose and ammonia represses glutaminase activity. GB-A-1 199 068 discloses a process for producing L-glutamic acid by culturing microorganisms in a culture medium containing acetic acid or a salt thereof as a main carbon source.

Cook W. R. et al.: "Occurence of an Inducible Glutaminase in Bacillus licheniformis", J. OF BACTERIOLOGY, Oct. (1981), Vol. 148, No. 1, pages 365 to 367 discloses the cultivation of B. licheniformis which leads to an increased activity of glutaminase by using glucose as the carbon source and additionally a nitrogen source.

### Disclosure of the Invention

The present inventors have assiduously conducted investigations to solve the problems, and have consequently found that the glutaminase production is rapidly improved by releasing catabolite repression during the incubation of a microorganism and as required, feeding a nitrogen source in the intermediate stage of the incubation. This finding has led to the completion of the invention.

The present invention provides the following.
(1) A process for producing a culture of a koji mold or a yeast capable of producing glutaminase, the culture having an increased glutaminase activity, characterised in that a carbon source is fed such that catabolite repression of said glutaminase is released or catabolite repression does not occur during incubation of a koji mold or a yeast capable of producing glutaminase, and a nitrogen source is fed in the intermediate stage of the incubation as required.
(2) The process according to (1), wherein the catabolite repression is released by feeding a carbon source either continuously or intermittently during the incubation.
(3) A culture produced by the process according to (1) and having a glutaminase activity increased to double or more in comparison with the activity provided through incubation, in a medium containing glucose at an initial concentration of 1.5 % (wt./vol.) without feeding a carbon source during the incubation.
(4) A process for producing hydrolysed protein, characterized in that protein is subjected to a reaction with the culture having an increased glutaminase activity according to (3) in the presence of proteolytic enzymes and either in the absence of sodium chloride or in the presence of sodium chloride at a concentration of 3 % (weight/volume) or less.
(5) The process for producing hydrolysed protein according to (4), wherein when protein is subjected to a reaction with the culture, the reaction is first conducted at a temperature range of from 15 to 39°C while conducting aeration and stirring, and then at a temperature range of from 40 to 60°C without the aeration.

The present invention is described in detail hereinafter.

Microorganisms used in the invention are koji molds or yeasts capable of producing glutaminase. Of these, koji molds are preferable. As koji molds, yellow koji molds typified by Aspergillus oryzae and Aspergillus sojae are preferable. Further, as yeasts, Bullera derxii is preferable.

The medium used in the invention is one in which the microorganisms can be grown, and it generally contains a carbon source, a nitrogen source and a cofactor.

When the carbon source in the medium is a carbon source which is rapidly assimilated by microorganisms, such as glucose, maltose, fructose and sucrose, it has to be fed such that the concentration is kept low during the incubation for the purpose of releasing the catabolite repression therewith. Specifically, the carbon source has to be fed continuously such that the concentration of the carbon source in the culture liquid is maintained at 0.5 % (weight/volume) or less, preferably 0.2 % (weight/volume) or less. When the carbon source is fed intermittently, it has to be fed such that the concentration of the carbon source in the culture liquid is maintained at 1.2 % (weight/volume) or less, preferably 0.5 % (weight/volume) or less. By the way, glucose may be in the form of a hydrolyzate of starch, and sucrose in the form of molasses.

Meanwhile, with respect to the carbon source which is relatively slowly assimilated by microorganisms, such as lactose, mannitol and sorbose, no catabolite repression occurs even when it is fed in a usual amount from the initial stage of the incubation. Accordingly, such a carbon source is added to a medium at a concentration of from 0.5 to 3.0 % (weight/volume), preferably from 1.0 to 2.0 % (weight/volume) in the initial stage of the incubation.

These carbon sources may be used either singly or in combination. In this manner, the catabolite repression during the incubation of microorganisms is released, making it possible to improve the glutaminase production and to produce the glutaminase in a high yield.

Preferable examples of the nitrogen source in the medium include inorganic nitrogen sources such as sodium nitrate, sodium nitrite, ammonium chloride and ammonium sulfate, organic nitrogen sources such as casamino acid, polypeptone, bacto-peptone, soybean protein, separated soybean protein, de-fatted soybean and casein, and amino acids such as sodium L-glutamate, sodium L-aspartate, L-proline, L-alanine, glycine and L-glutamine. Especially, such a nitrogen source is fed in the intermediate stage of the incubation whereby the glutaminase production can further be increased. The amount of the nitrogen source to be added is between 0.1 and 2.0 % (weight/volume), preferably between 0.5 and 1.0 % (weight/volume).

These nitrogen sources may be used either singly or in combination. When ammonium or glutamine is used as a nitrogen source, the glutaminase production is repressed at times, but the repression of the production can be avoided by adding the same in small portions.

Further, examples of the cofactor include magnesium sulfate 7-hydrate, disodium hydrogenphosphate, monosodium hydrogenphosphate, monopotassium hydrogenphosphate, dipotassium hydrogenphosphate, meat extract, potassium chloride and corn steep liquor. These are used selectively.

With respect to the amounts of these, it is most appropriate that, for example, magnesium sulfate is approximately 0.5 % (weight/volume), and monopotassium hydrogenphosphate is approximately 0.5 % (weight/volume).

Further, the incubation conditions in the initial stage may be ordinary aerobic incubation conditions. The pH is between 4.5 and 9.0, preferably between 5.5 and 8.5. The temperature is between 15 and 40 °C, preferably between 25 and 35 °C. Still further, the rate of agitation and the amount of aeration are not particularly limited so long as the aerobic incubation atmosphere can be maintained.

When the microorganisms are incubated according to the process of the invention, the glutaminase activity can be improved to from approximately 2 to 32 times in comparison with the ordinary process, and the glutaminase can be produced in a high yield. Therefore, the expression "increased glutaminase activity" in the present invention means that the activity is increased to double or more, preferably five times or more in comparison with the activity provided through incubation in a medium containing glucose at an initial concentration of 1.5 % (weight/volume) without feeding a carbon source during the incubation.

In addition, when seasonings are produced using a koj i mold, the process of the invention improves the amount of glutaminase produced in the culture liquid, making it possible to produce a high-quality product at good efficiency.

The thus-obtained microbial culture having the high glutaminase activity is used for the reaction with protein in the presence of proteolytic enzymes, whereby a hydrolyzate having a strong umami can be obtained.

The proteolytic enzymes used may be a commercial enzyme preparation, and may contain other enzymes such as a cell wall decomposition enzyme. Further, a purified enzyme can also be used. Especially when a koji mold is used as a microorganism for increasing the glutaminase activity, the koji mold itself produces proteolytic enzymes. Accordingly, the culture has both the glutaminase activity and the protease activity, and there is no need to add proteolytic enzymes from outside.

Examples of the protein which is subjected to a reaction with the microbial culture having the high glutaminase activity include soybeans, wheat, wheat gluten, corn meal, milk casein and fish meal. Further, de-fatted soybean, various proteins which have undergone processing such as puffing or solubilization, or separated proteins from these various protein starting materials are also available. Especially, proteins containing glutamine in a large amount, such as wheat gluten, are appropriate for being subjected to the reaction with the microbial culture having the high glutaminase activity.

The conditions under which the protein is subjected to the reaction with the microbial culture having the high glutaminase activity are described as follows. For example, it is advisable that the starting material at a concentration of from 0.2 to 50 %, preferably from 1 to 20 % is mixed with the microbial culture having the high glutaminase activity in the presence of the protease, and the mixture is reacted at from 5 to 60°C, preferably from 30 to 50°C for from 4 hours to 10 days, preferably from 10 hours to 5 days.

By the way, the protein starting material contains at times a carbohydrate such as starch which is hydrolyzed during the reaction to form reducing sugar such as glucose. When reducing sugar remains in the hydrolyzed protein formed, it causes browning. Accordingly, it is advisable to adjust the amount of reducing sugar to 5 % (weight/weight) or less, preferably, 3 % (weight/weight) or less, more preferably, 1.5 % (weight/weight) or less, based on the total solid content in the reaction product. Specifically, the amount of reducing sugar in the reaction product can be reduced by conducting the reaction first at a temperature range of from 15 to 39 °C, preferably, from 25 to 38°C, while conducting aeration and stirring, assimilating the reducing sugar, then conducting the reaction at a temperature range of from 40 to 60 °C, preferably, from 41 to 50°C, upon stopping the aeration, and thereafter completing the reaction. The reaction temperature is shifted when from 10 to 60 % of the overall reaction time passes from the start-up of the reaction.

The hydrolysis of the protein is conducted in the absence of sodium chloride or in the presence of sodium chloride at a low concentration of 3 % (weight/volume) or less. Since the activities of the protease and the glutaminase are sometimes inhibited by sodium chloride, the lower concentration of sodium chloride is desirable for improving the reaction rate. However, a small amount of ethanol or sodium chloride may be added during the reaction for the purpose of antisepsis.

After the completion of the reaction, insoluble matters such as unreacted starting proteins and cells can be removed by an ordinary separation method such as centrifugation or filtration. Further, as required, a liquid portion after solid-liquid separation may be concentrated through vacuum concentration or reverse osmosis. Especially when the pH is adjusted to an alkaline region and concentration is conducted in vacuo, the ammonia content in the reaction solution can be reduced. According to the inventors' findings, when wheat gluten having a high glutamine content is used as a starting material, the ammonia content in the hydrolyzed protein is higher than that in a product derived from another starting material, causing browning of the final product.

Accordingly, it is advisable to reduce the ammonia content in the hydrolyzed protein. The ammonia content can be adjusted such that the ammonia-type nitrogen content/total nitrogen content in the total solid content is 0.35 % (weight/weight) or less, preferably 0.15 % (weight/weight) or less.

Further, the concentrate can be pulverized or granulated through drying treatment such as freeze-drying, vacuum-drying or spray-drying. Thus, the hydrolyzed protein having the high content of monosodium glutamate and the strong seasoning property can be obtained without adding monosodium glutamate from outside.

The thus-obtained hydrolyzed protein is, unlike the hydrochloric acid hydrolyzate, free from monochlorohydroxypropanols and dichloropropanols. This is identified from the data of less than the detection limits by a method in which the substances are extracted with an extrelut column and the derivatives with phenylboric acid are detected by gas chromatography-mass spectrometry (GC-MS) (Ushijima, et al., Shohin Eiseigaku Zasshi, 36.3, 360 - 364, 1995). Incidentally, examples of monochlorohydroxypropanols include 3-chloro-1,2-propanediol and 2-chloro-1,3-propanediol, and examples of dichloropropanols include 1,3-dichloro-2-propanol and 2,3-dichloro-1-propanol.

The detection limits of these substances according to this method are 0.005 ppm in liquid samples and 0.05 ppm in powdery samples.

The hydrolyzed protein is added to various foods (wheat flour products, instant foods, agricultural, stockbreeding and marine processed products, dairy dishes, fats and oils, frozen foods, basic and mixed seasonings, confectionery, favorite beverages and the like) to enhance a umami and sweetness, to impart thickness and development, to impart mildness, to increase a flavor, to decrease a salty taste and a sour taste and to mask a queer taste. Especially, it is quite effective for (1) imparting sweetness, mildness and thickness in the presence of soy sauce, (2) masking a queer taste of meat product and enhancing a meat flavor, (3) enhancing a flavor in combination with pork, chicken, beef, seafood and vegetable extracts, and (4) enhancing a spicy taste or hot spicy taste in the presence of spices.

Since the hydrolyzed protein has a weak aroma, a light color and a high monomonosodium glutamate content, it can increase the above-mentioned effects and enhance umami without changing the original taste of foods or increasing the color thereof. Further, foods which are stronger in umami and kokumi (thickness, mouthfulness and continuity) can be provided by the combined use with umami ingredients such as nucleic acid, yeast extract or hydrolyzed proteins.

### Best Mode of Carrying Out the Invention

The invention is illustrated specifically by referring to the following Examples. However, the invention is not limited to these Examples.

Aspergillus oryzae (ATCC 11494) is described in the catalogue published by American Type Culture Collection (12301 Parklawn Drive, Rockville, Maryland 20852-1776, U.S.A.) and Aspergillus sojae (JCM 2250) and Bullera derxii(JCM 5280) in the catalogue published by Japan Collection of Microorganisms, The Institute of Physical and Chemical Research (RIKEN), (2-1, Hirosawa, Wako-shi, Saitama-ken) respectively. These can be supplined upon request.

The glutaminase activity in Examples was measured by the following method.

The glutaminase activity was measured by the modified method of Hartman et al. (Hartman, S. C.: J. Biol. Chem., 243, 853-863 (1968)) for determining the amount of γ-glutamylhydroxamic acid formed through an enzyme reaction in the presence of hydroxylamine. Specifically, 200 µl of a culture liquid were added to 1 ml of a mixed solution containing the reagent A (containing 0.4 M tris-aminomethane and 0.2 M hydroxylamine hydrochloride (pH 7.0)) and the reagent B (containing 100 mM L-glutamine and 20 mM reducing glutathione (pH 7.0)) in equal amounts, and the mixture was incubated at 37 °C for 1 hour. Then, for stopping of the reaction and color formation, 1 ml of a mixed solution containing (1) 3 N hydrochloric acid, (2) 12 % trichloroacetic acid and (3) a solution of 5 % ferric chloride 6-hydrate in 0.1 N hydrochloric acid in equal amounts was added thereto. The mixture was stirred, and the absorbance of the supernatant in this reaction solution was measured at 525 nm to find an enzymatic activity. The enzymatic activity to form 1 µg of γ-glutamylhydroxamic acid per minute under the above-mentioned conditions was defined as 1 unit (U).

### Example 1 (Production of a microbial culture having an increased glutaminase activity using Aspergillus oryzae)

Two liters of a medium (pH unadjusted) containing 1.5 % lactose or glucose, 1.5 % polypeptone, 0.5 % magnesium sulfate 7-hydrate, 0.25 % potassium chloride and 0.25 % monosodium hydrogenphosphate were charged into a jar fermenter having a total volume of 5 liters, and autoclaved. Then, Aspergillus oryzae (ATCC 11494) was inoculated therein in a usual manner. At this time, the koji mold is inoculated in any form, and it may be either spores or hyphae. Subsequently, the incubation was conducted at a temperature of 30 °C with a stirring rate of 600 rpm and an aeration rate of 1/2 vvm for 48 hours.

After the completion of the incubation, the glutaminase activity of the culture was measured. As a result, the amount of glutaminase produced in the culture liquid was 0.057 (u/ml) when glucose, which is rapidly assimilated, was the carbon source in the medium, whereas it was 0.185 (u/ml) when lactose, which is slowly assimilated, was the carbon source. Thus, the amount of glutaminase produced with lactose was more than 3 times that of glutaminase produced with glucose. That is, the use of lactose, the carbon source with the low rate of assimilation released the catabolite repression and improved the amount of glutaminase.

Further, when lactose was used as a carbon source, 1.0 % sodium L-glutamate 1-hydrate was added in the intermediate stage of the incubation and the incubation was continued. As a result, the amount of glutaminase produced was increased to 0.401 (u/ml).

As is clear from the results, the amount of glutaminase produced could be increased to approximately 7 times by using lactose as a carbon source and continuing the incubation with the addition of 1.0 % sodium L-glutamate 1-hydrate as a nitrogen source in the intermediate stage of the incubation.

### Example 2 (Production of a microbial culture having an increased glutaminase activity using Aspergillus oryzae)

Two liters of a medium (pH unadjusted) containing 1.5 % mannitol or sucrose, 1.5 % polypeptone, 0.5 % magnesium sulfate 7-hydrate, 0.25 % potassium chloride and 0.25 % monosodium hydrogenphosphate were charged into a jar fermenter having a total volume of 5 liters, and autoclaved. Then, Aspergillus oryzae (ATCC 11494) was inoculated therein in a usual manner. At this time, the koji mold is inoculated in any form, and it may be either spores or hyphae. Subsequently, the incubation was conducted at a temperature of 30°C with a stirring rate of 600 rpm and an aeration rate of 1/2 vvm for 48 hours.

After the completion of the incubation, the glutaminase activity of the culture was measured. As a result, the amount of glutaminase produced in the culture liquid was 0.011 (u/ml) when sucrose, which is rapidly assimilated, was the carbon source in the medium, whereas it was 0.148 (u/ml) when mannitol, which is slowly assimilated, was the carbon source. Thus, the amount of glutaminase produced with mannitol was more than 13 times that of glutaminase produced with sucrose. That is, the use of mannitol, the carbon source with the low rate of assimilation released the catabolite repression and improved the amount of glutaminase.

Further, when mannitol was used as a carbon source, 1.0 % sodium L-glutamate 1-hydrate was added in the intermediate stage of the incubation the incubation was continued. As a result, the amount of glutaminase produced was increased to 0.331 (u/ml).

As is clear from the results, the amount of glutaminase produced could be increased to approximately 30 times by using mannitol as a carbon source and continuing the incubation with the addition of 1.0 % sodium L-glutamate 1-hydrate as a nitrogen source in the intermediate stage of the incubation.

### Example 3 (Production of a microbial culture having an increased glutaminase activity using Aspergillus sojae)

Two liters of the medium A (containing 1.5 % glucose, 1.5 % polypeptone, 0.5 % magnesium sulfate 7-hydrate, 0.25 % potassium chloride and 0.25 % monosodium hydrogenphosphate (pH unadjusted)) or the medium B (containing 1.5 % polypeptone, 0.5 % magnesium sulfate 7-hydrate, 0.25 % potassium chloride and 0.25 % monosodium hydrogenphosphate (pH unadjusted)) were charged into a jar fermenter having a total volume of 5 liters, and autoclaved. Then, Aspergillus sojae (JCM 2250) was inoculated therein in a usual manner. At this time, the koji mold may be inoculated in the form of spores or hyphae. Subsequently, the incubation was conducted at a temperature of 30 °C with a stirring rate of 600 rpm and an aeration rate of 1/2 vvm for 34 hours.

With respect to the jar fermenter in which the fermentation was started in the medium B, a total of two jar fermenters were arranged, namely, one in which glucose was continuously added to maintain the concentration at 0.2 % or less and one in which glucose was continuously added to maintain the concentration at 0.2 % or less and 1.0 % sodium L-glutamate 1-hydrate as a nitrogen source was added on hour 17 from the start-up of the incubation which was the intermediate period of the incubation.

After the completion of the incubation, the glutaminase activity of the culture was measured. As a result, the amount of glutaminase produced in the culture liquid of the medium A was 0.002 (u/ml), the amount of glutaminase produced in the culture liquid of the medium B with a continuous addition of glucose at the concentration of 0.2 % or less was 0.022 (u/ml), and the amount of glutaminase produced in the culture liquid of the medium B with a continuous addition of glucose at the concentration of 0.2 % or less and an addition of 1.0 % sodium L-glutamate 1-hydrate on hour 17 of the incubation was 0.064 (u/ml).

As is clear from the results, the amount of glutaminase produced by continuously adding glucose at the low concentration to release catabolite repression was improved to approximately 11 times in comparison with the amount of glutaminase produced by conducting the incubation with adding glucose, which is rapidly assimilated, at the high concentration from the start-up of the incubation. Further, the amount of glutaminase produced by the koji mold was improved to 32 times through the incubation upon adding sodium L-glutamate 1-hydrate in the intermediate stage of the incubation.

### Example 4 (Production of a microbial culture having an increased glutaminase activity using a yeast)

Two liters of the medium A (containing 1.5 % glucose, 0.5 % bacto-peptone, 0.3 % malt extract and 0.3 % yeast extract (pH unadjusted)) or the medium B (containing 0.5 % bacto-peptone, 0.3 % malt extract and 0.3 % yeast extract (pH unadjusted)) were charged into a jar fermenter having a total volume of 5 liters, and autoclaved. Then, Bullera derxii (JCM 5280) was inoculated therein in a usual manner. Subsequently, the incubation was conducted at a temperature of 25°C with a stirring rate of 800 rpm and an aeration rate of 1/2 vvm for 55 hours.

With respect to the jar fermenter in which the fermentation was started in the medium B, a total of two jar fermenters were arranged, namely, one in which glucose was continuously added to maintain the concentration at 0.05 % or less and one in which glucose was continuously added to maintain the concentration at 0.05 % or less and 1.0 % sodium L-glutamate 1-hydrate as a nitrogen source was added on hour 24 of the incubation which was the intermediate period of the incubation.

After the completion of the incubation, the glutaminase activity of the culture was measured. As a result, the amount of glutaminase produced in the culture liquid of the medium A was 0.121 (u/ml), the amount of glutaminase produced in the culture liquid of the medium B with a continuous addition of glucose was 0.252 (u/ml), and the amount of glutaminase produced in the culture liquid of the medium B with a continuous addition of glucose and an addition of 1.0 % sodium L-glutamate 1-hydrate on hour 24 of the incubation was 0.486 (u/ml):

As is clear from the results, the amount of glutaminase produced by continuously adding glucose at the low concentration to release catabolite repression was improved to approximately 2.1 times in comparison with the amount of glutaminase produced by conducting the incubation upon adding glucose having the high rate of assimilation at the high concentration from the start-up of the incubation. Further, the amount of glutaminase was improved to 4 times by feeding sodium L-glutamate 1-hydrate in the intermediate stage of the incubation.

### Example 5 (Liberation of glutamic acid from wheat gluten)

The following procedure was sterilely conducted. Twenty milliliters of a solution of 5 % wheat gluten (trade name: "Ajipuron G2", supplied by Ajinomoto Co., Inc.) which had been autoclaved were mixed with 10 ml of the culture of Aspergillus oryzae (ATCC 11494) obtained by using mannitol as a carbon source as described in Example 2. The mixture was reacted at 40 °C for 10 hours, 24 hours, 4 days or 10 days.

For comparison, the same procedure was conducted using 10 ml of the culture of Aspergillus oryzae obtained by using sucrose as a carbon source as described in Example 2.

With respect to the supernatants of these reaction products, the total nitrogen content (T-N) and the content of glutamic acid released were measured. The glutamic acid content was measured by the enzymatic method, and the total nitrogen content by the Kjeldahl method. The analytical values and the rate of liberation of glutamic acid (glutamic acid content/total nitrogen content) obtained from the glutamic acid content and the total nitrogen content are shown in Table 1.

As shown in Table 1, the rate of liberation of glutamic acid in the hydrolyzed wheat gluten obtained with the culture of Aspergillus oryzae when using mannitol as a carbon source was clearly higher than that when using sucrose as a carbon source. Further, the hydrolyzate exhibited a strong taste.

**Table 1. Comparison with respect to a rate of liberation of glutamic acid in hydrolyzate liquid of wheat gluten**

| Carbon source In the medium | Reaction time | Glutamic acid content (g/dl) | Total nitrogen content (g/dl) | Rate of liberation of glutamic acid |
|---|---|---|---|---|
| Sucrose | 10 hours | 0.10 | 0.46 | 0.22 |
| | 24 hours | 0.14 | 0.46 | 0.32 |
| | 4 days | 0.23 | 0.47 | 0.48 |
| | 10 days | 0.42 | 0.47 | 0.89 |
| Mannitol | 10 hours | 0.54 | 0.46 | 1.18 |
| | 24 hours | 0.65 | 0.46 | 1.41 |
| | 4 days | 0.80 | 0.47 | 1.70 |
| | 10 days | 0.87 | 0.47 | 1.85 |

### Example 6 (Production of hydrolyzed wheat gluten)

The following incubation and hydrolysis were conducted sterilely. Two liters of a medium (pH unadjusted) containing 1.5 % separated soybean protein (trade name: "Ajipuron E3", supplied by Ajinomoto Co., Inc.), 0.5 % magnesium sulfate 7-hydrate, 0.25 % potassium chloride and 0.25 % sodium hydrogenphosphate were charged into a jar fermenter having a total volume of 5 liters, and autoclaved. Aspergillus oryzae (ATCC 11494) was inoculated therein in a usual manner. The incubation was conducted at a temperature of 30 °C with a stirring rate of 600 rpm and an aeration rate of 1/2 vvm. During the incubation, the glucose solution was added intermittently in four divided portions such that the total amount of glucose added was 1.5 % (weight/volume) and the glucose concentration in the culture liquid did not exceed 0.5 %. Further, the incubation was continued by adding 0.5 % sodium L-glutamate in the intermediate stage of the incubation, and the incubation was completed in 48 hours.

One liter of this culture and 2 liters of a 5 % wheat gluten (trade name: "Ajipuron G2", supplied by Ajinomoto Co., Inc.) dispersion were autoclaved, and mixed. The mixture was reacted in a small-sized jar fermenter at 35 °C for 8 hours while conducting aeration and stirring. Then, the aeration was stopped, and the hydrolysis reaction was conducted at 45 °C for 16 hours.

This hydrolyzate was subjected to solid-liquid separation using a Buchner funnel. The resulting product was concentrated with sodium hydroxide in the same molar amount as ammonia of the filtrate by means of an evaporator to remove ammonia. The concentrate was adjusted to pH 7.0 with the addition of hydrochloric acid, and heat-sterilized at 80 °C for 20 minutes. The product was cooled, and the precipitate was withdrawn using a Buchner funnel, and spray-dried using a disk-type spray drier to obtain a spray-dried product.

The total nitrogen content, the ammonia-type nitrogen content, the sodium chloride content, the free amino acid content and the reducing sugar content of the spray-dried product were measured. The sodium chloride content was calculated from the chloride content, and the free amino acid content was measured by the amino acid analysis. The reducing sugar content was measured by the Somogyi-Nelson method in terms of the glucose content. These analytical values are shown in Tables 2 and 3.

As shown in Table 3, hydrolyzed protein with the strong seasoning power containing 29.9 % (weight/weight) of glutamic acid (as monosodium glutamate) and having the total nitrogen content of 9.1 % (weight/weight) and the total amino acid content of 55.4 % (weight/weight) could be produced as the spray-dried product by hydrolyzing wheat gluten using the culture of Aspergillus oryzae having the high glutaminase activity.

**Table 2. Free amino acid content and rate of liberation in a spray-dried product**

| Amino acid | Amount liberated (g/100 g) | Rate of Liberation (g/g·N) |
|---|---|---|
| Asp | 2.46 | 0.27 |
| Thr | 1.72 | 0.19 |
| Ser | 2.74 | 0.30 |
| Glu | 23.56 | 2.56 |
| Pro | 5.14 | 0.56 |
| Gly | 2.05 | 0.23 |
| Ala | 2.01 | 0.22 |
| Cys | 0.39 | 0.04 |
| Val | 2.62 | 0.29 |
| Met | 0.87 | 0.10 |
| Ile | 2.38 | 0.26 |
| Leu | 4.39 | 0.48 |
| Tyr | 0.62 | 0.07 |
| Phe | 2.02 | 0.22 |
| Lys | 1.26 | 0.14 |
| His | 1.03 | 0.11 |
| Arg | 0.17 | 0.02 |
| Total | 55.44 | 6.09 |

**Table 3. Analyzed values of a spray-dried product**

| Analysis items | Content (g/100 g) | Content based on nitrogen (g/g·N) |
|---|---|---|
| Glutamic acid* | 29.9 | 2.59 |
| Total nitrogen | 9.12 | 1.00 |
| Total free amino acid | 55.4 | 6.09 |
| Sodium chloride | 4.37 | 0.48 |
| Ammonia-type nitrogen | 0.48 | 0.053 |
| Reducing sugar | 1.33 | 0.149 |

| | | |
|---|---|---|
| *as monosodium glutamate | | |

### Industrial Applicability

The invention provides a microbial culture having an increased glutaminase activity. The use of this microbial culture can produce hydrolyzed protein at good efficiency. Since the resulting hydrolyzed protein has a high glutamic acid content, it exhibits a strong seasoning power, and is quite valuable as a seasoning.

## Claims

1. A process for producing a culture of a koji mold or a yeast capable of producing glutaminase, the culture having an increased glutaminase activity, **characterised in that** a carbon source is fed such that catabolite repression of said glutaminase is released or catabolite repression does not occur during incubation of a koji mold or a yeast capable of producing glutaminase, and a nitrogen source is fed in the intermediate stage of the incubation as required.

2. The process according to claim 1, wherein the catabolite repression is released by feeding a carbon source either continuously or intermittently during the incubation.

3. A culture produced by the process according to claim 1 and having a glutaminase activity increased to double or more in comparison with the activity provided through incubation, in a medium containing glucose at an initial concentration of 1.5 % (wt./vol.) without feeding a carbon source during the incubation.

4. A process for producing hydrolysed protein, **characterized in that** protein is subjected to a reaction with the culture having an increased glutaminase activity according to claim 3 in the presence of proteolytic enzymes and either in the absence of sodium chloride or in the presence of sodium chloride at a concentration of 3 % (weight/volume) or less.

5. The process for producing hydrolysed protein according to claim 4, wherein when protein is subjected to a reaction with the culture, the reaction is first conducted at a temperature range of from 15 to 39°C while conducting aeration and stirring, and then at a temperature range of from 40 to 60°C without the aeration.

## Patentansprüche

1. Verfahren zur Herstellung einer Koji-Mold-Kultur oder einer Hefekultur, befähigt zur Herstellung von Glutaminase, wobei die Kultur eine erhöhte Glutaminaseaktivität aufweist, **dadurch gekennzeichnet, dass** eine Kohlenstoffquelle derart zugeführt wird, dass die Katabolitrepression der Glutaminase herabgesetzt wird, oder eine Katabolitrepression während der Inkubation eines Koji-Mold oder einer Hefe, befähigt zur Herstellung von Glutaminase, nicht auftritt, und nach Bedarf eine Stickstoffquelle in der Zwischenstufe der Inkubation zugeführt wird.

2. Verfahren nach Anspruch 1, wobei die Katabolitrepression durch Zuführen einer Kohlenstoffquelle entweder kontinuierlich oder in Abständen während der Inkubation herabgesetzt wird.

3. Kultur, hergestellt durch das Verfahren nach Anspruch 1 und mit einer Glutaminaseaktivität, welche im Vergleich mit der Aktivität, die durch Inkubation bereitgestellt wird, auf das doppelte oder höher erhöht ist, in einem Medium, welches Glukose bei einer Anfangskonzentration von 1,5 % (Gew./Vol.) enthält, ohne während der Inkubation eine Kohlenstoffquelle zuzuführen.

4. Verfahren zur Herstellung von hydrolisiertem Protein, **dadurch gekennzeichnet, dass** Protein einer Reaktion mit der Kultur mit einer erhöhten Glutaminaseaktivität nach Anspruch 3 in Gegenwart proteolytischer Enzyme und entweder in Abwesenheit von Natriumchlorid oder in Gegenwart von Natriumchlorid in einer Konzentration von 3 % (Gew./Vol.) oder weniger unterzogen wird.

5. Verfahren zur Herstellung von hydrolisiertem Protein nach Anspruch 4, wobei, wenn das Protein einer Reaktion mit der Kultur unterzogen wird, die Reaktion zunächst bei einem Temperaturbereich von 15 bis 39 °C unter Belüftung und Rühren durchgeführt wird, und anschließend bei einem Temperaturbereich von 40 bis 60 °C ohne die Belüftung.

## Revendications

1. Procédé de production d'une culture de moisissure koji ou de levure capable de produire une glutaminase, la culture ayant une activité glutaminase accrue, **caractérisé par** un apport en source de carbone tel que la répression catabolique de ladite glutaminase est libérée ou que la répression catabolique ne survient pas pendant l'incubation d'une moisissure koji ou d'une levure capable de produire une glutaminase et par un apport, comme nécessaire, en source d'azote dans l'étape intermédiaire de l'incubation.

2. Procédé selon la revendication 1, dans lequel la répression catabolique est libérée par un apport en source de carbone soit continu soit intermittent pendant l'incubation.

3. Culture produite par le procédé selon la revendication 1 et ayant une activité glutaminase multipliée par deux ou plus par comparaison avec l'activité fournie par l'incubation, dans un milieu contenant du glucose à une concentration initiale de 1,5 % (p/vol.) sans apport en source de carbone pendant l'incubation.

4. Procédé de production d'une protéine hydrolysée, **caractérisé en ce que** la protéine est soumise à une réaction avec la culture ayant une activité glutaminase accrue selon la revendication 3 en présence d'enzymes protéolytiques et soit en l'absence de chlorure de sodium, soit en présence de chlorure de sodium à une concentration de 3 % (poids/volume) ou moins.

5. Procédé de production d'une protéine hydrolysée selon la revendication 4 dans lequel, quand la protéine est soumise à une réaction avec la culture, la réaction est d'abord mise en oeuvre dans une plage de températures de 15 à 39°C sous aération et agitation, puis dans une plage de températures de 40 à 60°C sans aération.
